# EUROPEAN PATENT SPECIFICATION

(11) **EP 2 863 816 B1**
(45) Date of publication and mention of the grant of the patent: **04.04.2018**
(21) Application number: 13734258.0
(22) Date of filing: 21.06.2013
(51) Int. Cl.: A61B 17/70, A61B 17/80, A61B 17/86

(54) **LOW PROFILE BONE STABILIZATION SYSTEMS**
KNOCHENSTABILISIERUNGSSYSTEME MIT NIEDRIGEM PROFIL
SYSTÈME DE STABILISATION D'OS À PROFIL BAS

(30) Priority: 21.06.2012 US 201261662587 P
(43) Date of publication of application: 29.04.2015
(73) Proprietor: Aesculap AG, 78532 Tuttlingen (DE)
(72) Inventor: PRAJAPATI, Mohit, Moorestown, New Jersey 08057 (US); COLE, Jeffrey, Macungie, PA 18062 (US)
(74) Representative: Hoeger, Stellrecht & Partner Patentanwälte mbB
(86) International application number: PCT/US2013/046972
(87) International publication number: WO 2013/192489

(56) References cited:
- WO-A1-03/068088
- US-A1- 2005 192 572
- US-A1- 2006 052 783
- US-A1- 2006 264 936
- US-A1- 2008 312 692
- US-A1- 2009 069 849
- US-A1- 2010 094 345
- US-A1- 2010 211 116
- US-A1- 2012 226 316

## Description

The present invention relates to low profile spinal stabilization systems as defined in the preamble of claim 1. Preferred embodiments are set forth in the dependent claims.

### BACKGROUND

Bone stabilization systems, such as bone screw and rod assemblies, can be used as an adjunct to spinal fusion surgery and correction of spinal deformities. In such systems, two or more bone screws provide a means for anchoring a rod or other type of elongated fixation member. The rod fixates adjacent vertebral bodies, for example the vertebrae at L4 and L5. Bone screw and rod assemblies provide stability and prevent motion at the segments that are being fused. The list of indications for bone screw and rod assemblies include but are not limited to:
∘ Spondylolithesis;
∘ Fracture;
∘ Deformity;
∘ Tumor;
∘ Pseudarthrosis; and
∘ Spinal Stenosis.

Conventional bone stabilization systems feature some form of locking mechanism to lock the rod to the bone screw. For example, the locking mechanism may feature a set screw that is screwed down into a rod receiver body associated with the screw head. In the locked condition, the set screw locks the rod against the screw head. This arrangement requires a portion of each screw head to project above the bone into which the screw is anchored so that: (1) the rod can attach to a part of the screw, and (2) the locking mechanism can attach to the screw. As such, locking mechanisms in conventional bone stabilization systems extend above the bone into which the bone stabilization system is anchored, and project into tissue above the bone. Displacement of tissue above the bone can cause tissue injury during and after surgery.

When performing posterior thoracolumbar (TL) or cervical stabilization, a bone screw and rod assembly is often installed on both sides of the midline of the spine. Bone screw and rod assemblies can be unstable, and sometimes require "cross connectors" interconnected between the bone screw and rod assemblies to provide additional torsion stability. This need is very apparent in longer constructs that are greater than two levels. It can be challenging to attach cross connectors between longer bone screw and rod assemblies because the rods are often non-parallel and/or non-planar. Non-parallel and non-planar rods present a problem because many known rod connectors require the two spinal rods to be more or less parallel and co-planar.

Orthopedic anchor assemblies are known from US 2010/0211116 A1. Systems and methods for bone fixation are described in US 2006/0264936 A1. US 2006/0052783 A1 discloses polyaxial devices for spine stabilization during osteosynthesis. Posterior polyaxial systems for the spine are known from WO 03/068088 A1. Medialised rod pedicle screw assemblies are described in US 2005/0192572 A1. US 2009/0069849 A1 discloses dynamic screw systems. Transconnectors are known from US 2010/0094345 A1. Transverse connectors are described in US 2012/0226316 A1. US 2008/0312692 A1 discloses multi-level spinal stabilization systems.

### SUMMARY

The present invention is defined by the subject matter of claims 1. Preferred embodiments of the invention are defined in the dependent claims.

Low profile bone stabilization systems in accordance with the invention resolve many drawbacks and challenges that are encountered when using conventional bone stabilization systems and cross connectors.

In a first embodiment, a low profile spinal stabilization system includes:
an elongated fixation element having a first socket at a first end of the elongated fixation element and a second socket at a second end of the elongated fixation element;
a first anchor element having a spherical end configured for insertion into the first socket of the elongated fixation element, the spherical end of the first anchor element having a plurality of petals separated by a plurality of radially arranged slots;
a second anchor element having a spherical end configured for insertion into the second socket of the elongated fixation element, the spherical end of the second anchor element having a plurality of petals separated by a plurality of radially arranged slots;
a first locking element configured for insertion into the spherical end of the first anchor element to lock the spherical end of the first anchor element inside the first socket of the elongated fixation element when the spherical end of the first anchor element is positioned inside the first socket; and
a second locking element configured for insertion into the spherical end of the second anchor element to lock the spherical end of the second anchor element inside the second socket of the elongated fixation element when the spherical end of the second anchor element is positioned inside the second socket.

At least one of the first anchor element and second anchor element can include a bone screw. Alternatively, at least one of the first anchor element and second anchor element can include an adapter for attaching the elongated fixation element to a bone screw assembly. In the latter case, the adapter can include a threaded end opposite the spherical end, the threaded end configured for anchoring a rod in the bone screw assembly.

In another embodiment, a low profile spinal stabilization system includes:
a first bone screw having a spherical head having a plurality of petals separated by a plurality of radially arranged slots;
a second bone screw having a spherical head having a plurality of petals separated from one another by a plurality of radially arranged slots;
an elongated fixation rod having a first socket extending through a first end of the elongated fixation rod and a second socket extending through a second end of the fixation rod, the first socket configured to receive the spherical head of the first bone screw, and the second socket configured to receive the spherical head of the second bone screw;
a first locking element configured for insertion between the petals of the first bone screw and into the spherical head of the first bone screw to radially expand the petals into a locked condition when the spherical head of the first bone screw is positioned inside the first socket; and
a second locking element configured for insertion between the petals of the second bone screw and into the spherical head of the second bone screw to radially expand the petals into a locked condition when the spherical head of the second bone screw is positioned inside the second socket.

The first locking element can be axially displaceable in the spherical head of the first bone screw between an unlocking position, in which the petals of the first bone screw are expanded radially outwardly and have a first outer diameter, and a locking position, in which the petals of the first bone screw are expanded further radially outwardly and have a second outer diameter, the second outer diameter greater than the first outer diameter.

The first bone screw can be polyaxially rotatable inside the first socket when the first bone screw is inserted into the first socket, and when the first locking element is inserted into the spherical head of the first bone screw in the unlocking position. In addition, the first bone screw can be fixed relative to the first socket when the first bone screw is inserted into the first socket, and when the first locking element is inserted into the spherical head of the first bone screw in the locking position.

At least one of the first socket and second socket can include a spherical inner wall that conforms to the spherical head of at least one of the first bone screw and second bone screw. At least one of the first socket and the second socket can also be cylindrical.

At least one of the first socket and second socket of the elongated fixation rod can be configured to receive substantially all of the spherical head of at least one of the first bone screw and second bone screw, and configured to receive substantially all of at least one of the first locking element and second locking element.

The first socket can include a bore, the bore having a first end, a second end opposite the first end, and an inner diameter that is greatest at a section of the bore located intermediate the first end and the second end. The inner diameter of the bore can be largest at the midsection of the bore.

At least one of the first bone screw and the second bone screw includes a shank. The spherical head of the at least one of the first bone screw and the second bone screw can be formed of a first material, and the shank can be formed of a second material. The first material can be more resilient and flexible than the second material.

In another embodiment, a low profile spinal stabilization system for use with a pedicle screw system that comprises a first bone screw assembly having a first bone screw and a first rod, and a second bone screw assembly having a second bone screw and a second rod, includes:
an elongated fixation element having a first socket at a first end of the elongated fixation element and a second socket at a second end of the elongated fixation element;
a first anchor element having a first end configured for anchoring the first rod in the first bone screw assembly, and a second end opposite the first end configured for insertion into the first socket of the elongated fixation element;
a second anchor element having a first end configured for anchoring the second rod in the second bone screw assembly, and a second end opposite the first end configured for insertion into the second socket of the elongated fixation element;
a first locking element configured for insertion into the second end of the first anchor element to lock the second end of the first anchor element inside the first socket of the elongated fixation element when the second end of the first anchor element is positioned inside the first socket; and
a second locking element configured for insertion into the second end of the second anchor element to lock the second end of the second anchor element inside the second socket of the elongated fixation element when the second end of the second anchor element is positioned inside the second socket.

The second end of each anchor element can include a plurality of petals separated from one another by a plurality of radially arranged slots.

The first locking element can be configured for insertion between the petals of the first anchor element to radially expand the petals into a locked condition when the second end of the first anchor element is positioned inside the first socket. Similarly, the second locking element can be configured for insertion between the petals of the second anchor element to radially expand the petals into a locked condition when the second end of the second anchor element is positioned inside the second socket.

The first ends of the first and second anchor elements can include threads. The second ends of the first and second anchor elements can be spherical.

### BRIEF DESCRIPTION OF THE DRAWINGS

The foregoing summary and the following detailed description will be better understood in conjunction with the drawing figures that illustrate non-limiting examples, of which:
Figure 1 is a partially exploded perspective view of a low profile stabilization system in accordance with one embodiment of the invention;
Figure 2 is a cross-section view of the low profile stabilization system of Figure 1, taken through line 2-2 in Figure 1;
Figure 3 is a partially exploded perspective view of the low profile stabilization system of Figure 1, shown with a known rod receiver body to illustrate the difference in height between the known rod receiver body and the system components;
Figure 4 is a perspective view of a low profile stabilization system in accordance with another embodiment of the invention;
Figure 5 is a partially exploded perspective view of the low profile stabilization system of Figure 4;
Figure 6 is a partially exploded cross-section view of the low profile stabilization system of Figure 4;
Figure 7 is a perspective view of a low profile stabilization system in accordance with another embodiment which is not in accordance with the present invention; and
Figure 8 is a partially exploded perspective view of the low profile stabilization system of Figure 7.

### DETAILED DESCRIPTION

Although the invention is described and illustrated herein with reference to specific embodiments, the invention is not intended to be limited to the embodiments shown. The embodiments represent examples only. Various modifications may be made in the details within the scope and range of equivalents of the claims without departing from the invention.

The following description contains different examples of bone stabilization systems that address drawbacks encountered with conventional bone stabilization systems. The bone stabilization systems will be described in the context of how they can be used for the purpose of spinal stabilization. Nevertheless, the bone stabilization systems and their components may have applications in areas of the body other than the spine. Therefore, bone stabilization systems in accordance with the invention are not limited to spinal stabilization.

Bone stabilization systems in accordance with preferred embodiments include screw heads, rods, plates, locking mechanisms and/or other components that have low profiles in the assembled condition. The term "low profile", as used herein, means an assembly that extends only a small distance above the bone into which the assembly is anchored, minimizing the amount of tissue above the bone that is displaced and possibly damaged during and after surgery.

To provide a low profile assembly, preferred systems in accordance with the invention may include an alternative screw and rod relationship. The rods are not inserted into the screw heads, as typically seen in conventional systems. Instead, the screw heads are inserted into the rods. The entire screw head and its internal locking mechanism are consolidated in a compact arrangement inside the rod. This compact arrangement provides a low profile that is significantly smaller in height than the profiles of conventional bone stabilization systems.

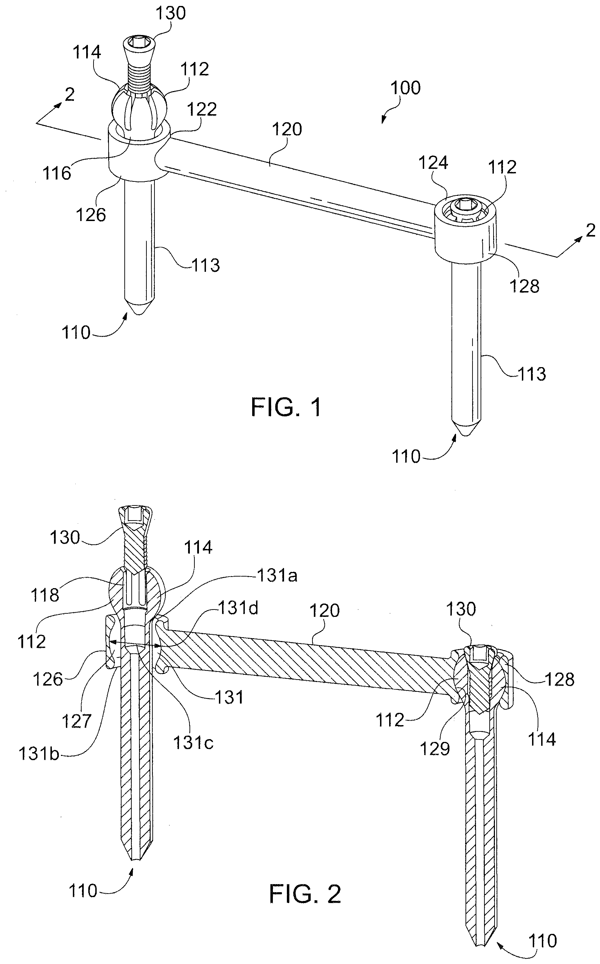

Referring to Figures 1-3, a low profile spinal stabilization system 100 is shown in accordance with one exemplary embodiment. System 100 includes a pair of bone screws 110 interconnected by an elongated fixation element in the form of a rod 120. Rod 120 is secured to bone screws 110 by a pair of locking elements in the form of locking screws 130.

System 100 illustrates one example of the alternative screw and rod relationship described above. Rod 120 is not inserted into the head of screws 110. Rather, the heads of screws 110 are inserted into rod 120. Each bone screw 110 has a rounded head 112. In particular, each head 112 is spherical. Each head 112 has a plurality of sections or petals 114 separated from one another by a plurality of radially arranged slots 116, and an axial bore 118 extending between the petals. Rod 120 includes a first end 122 featuring a first socket 126 and a second end 124 featuring a second socket 128. First socket 126 is configured to receive head 112 of one of the bone screws and second socket 128 is configured to receive the head of the other of the bone screws.

First socket 126 includes a spherical inner wall 127, and second socket 128 includes a spherical inner wall 129. First socket 126 includes a bore 131 that has a first end 131a, a second end 131b opposite the first end, and an inner diameter 131c that is greatest at a section of the bore located intermediate the first end and the second end. More specifically, inner diameter 131c is largest at the midsection 131d of bore 131. Second socket 128 has an identical geometry.

Petals 114 are preferably formed of a resilient flexible material. As such, petals 114 are displaceable between a relaxed condition and a flexed condition. In the relaxed condition, the petals are in their normal position, not displaced by external force. In the flexed condition, the petals are displaced radially outwardly under stored energy when an outwardly directed force is applied to the petals. When outwardly directed force is removed from petals 114, the stored energy in the petals returns the petals to their normal positions in the relaxed condition.

In some embodiments, the head of the bone screw is formed of a material that is more resilient and flexible than the material that forms other parts of the bone screw. For example, each bone screw 110 includes a head 112 and a threaded shank 113 that extends from the head. The head 112 of each bone screw 110 is formed of a first material, and the shank 113 of each bone screw is formed of a second material. The first material that forms each head 112 is more resilient and flexible than the material that forms each shank 113. This allows the heads 112 to more readily expand and engage the inner walls 127 and 129 of the first socket 126 and second socket 128, respectively, while the shanks 113 are comparatively more rigid.

Each locking screw 130 is configured for insertion into a bore 118 of one of the bone screws 110, so as to extend into the head 112 between the petals 114. When a locking screw 130 is inserted into the head 112 of a bone screw 110, the locking screw bears against the inner surface of each petal 114 and pushes the petal radially outwardly from the relaxed condition to the flexed condition. This preferably expands all of the petals at the same time. When head 112 is positioned in one of the sockets in the rod, advancement of the locking screw 130 into the head expands the petals radially outwardly until the petals are compressed against the interior of the socket. This compressed state immobilizes the screw head in a locked condition in the rod.

Figures 1 and 2 illustrate different components in the relaxed condition, flexed condition and locked condition. Bone screw 110 on the left side (or exploded portion) of the Figures is shown with locking element 130 removed from the screw head 112. Therefore, the petals 114 are in the relaxed condition on the left side of the Figures. In contrast, bone screw 110 on the right side of the Figures is shown with a locking element 130 inserted into the screw head 112. As such, petals 114 on this bone screw are expanded radially outwardly in the flexed condition. The bone screw 110 on the right has its head 112 inserted in second socket 128. Therefore, petals 114 are compressed against the interior of second socket 128, immobilizing the head of the screw in the locked condition.

Each locking element 130 is axially displaceable in the head 112 of a bone screw 110 between an unlocking position, in which the petals 114 of the bone screw are not expanded radially outwardly and have a first outer diameter (or first cross sectional dimension), and a locking position, in which the petals of the bone screw are expanded radially outwardly and have a second outer diameter (or second cross sectional dimension), the second outer diameter being greater than the first outer diameter. Each bone screw head 112 is polyaxially rotatable inside one of the sockets 126, 128 when the bone screw is inserted into the socket and a locking element 130 is inserted in the head in the unlocking position. Bone screw heads 112 are fixed relative to sockets 126, 128 when the bone screws are inserted into the sockets and the locking elements are inserted in the heads in the locking positions.

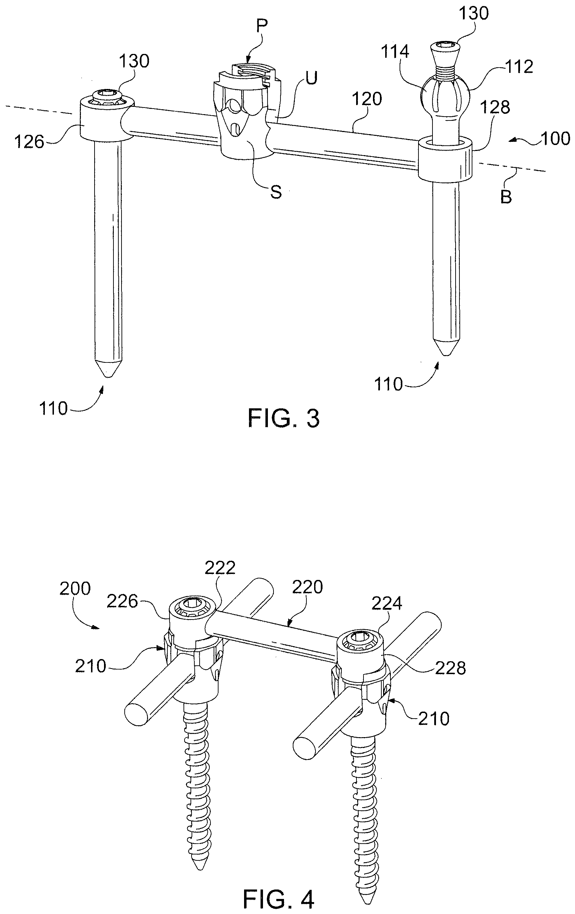

Figure 3 illustrates the difference in profile between system 100 and a known pedicle screw component P. Sockets 126, 128 and component P are shown in an aligned arrangement as they would be positioned above a bone surface, the bone surface being schematically represented by dashed line B. When system 100 is assembled, as shown on the left side, the system has a much lower vertical profile, i.e. a smaller height above bone surface B, than component P. This is due in large part to the interrelationship between the bone screws 110 and rod 120, in which the bone screw head 112 and locking screw 130 are nested inside the rod. In this nested arrangement, the height of screw head 112 coincides with the height of rod 120. Almost all of the length of each locking screw 130 is contained inside the head of a bone screws and inside a socket. Therefore, the vertical profile or height of the three nested components substantially overlap one another, minimizing the overall vertical profile or height of the assembly above the bone surface B.

In contrast to system 100, component P does not offer a consolidated and compact arrangement. Component P would not be assembled with the rod as shown, but would actually receive the rod at a relatively higher position inside the U-shaped channels U. As such, the entire rod would be positioned higher above bone surface B. In addition, U-shaped channels U receive the rod above a seat portion S that would contain the bone screw head. As such, the full vertical profile (i.e. cross section) of the rod would be positioned on top of the screw head. Therefore, the height of component P, at a minimum, must be the combined height of the screw head and the rod in order to contain both. Component P also has a threaded portion T to receive a locking nut above where the rod sits. Threaded portion T adds additional height to component P. Consequently, the vertical profile or height of a stabilization system utilizing component P will be significantly greater than the vertical profile or height of system 100. As stated above, a stabilization system utilizing component P would also position a rod higher above bone surface B than system 100.

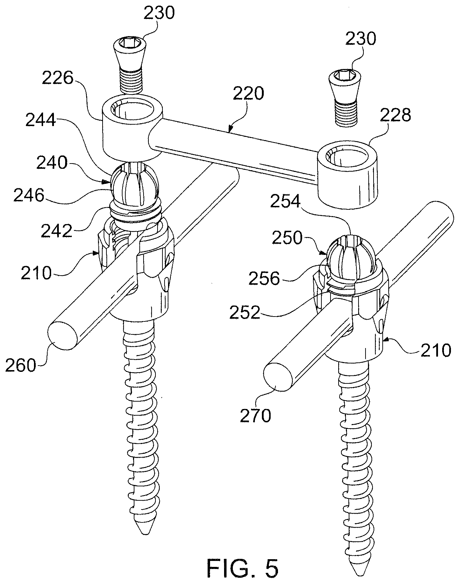

Referring to Figures 4-6, a low profile spinal stabilization system 200 is shown in accordance with another exemplary embodiment. System 200 includes a pair of bone screw assemblies 210 interconnected by an elongated fixation element in the form of a head to head connecting rod or cross connector 220. Cross connector 220 includes a first socket 226 at a first end 222 and a second socket 228 at a second end 224.

A first anchor element 240 includes a threaded end 242 and a rounded end 244 opposite the threaded end. Threaded end 242 is configured for anchoring a first rod 260 in one of the bone screw assemblies 210. Rounded end 244 is configured for insertion into first socket 226 of cross connector 220. A second anchor element 250 includes a threaded end 252 and a rounded end 254 opposite the threaded end. Threaded end 252 is configured for anchoring a second rod 270 in the other of the bone screw assemblies 210. Rounded end 254 is configured for insertion into second socket 228 of cross connector 220.

Rounded end 244 includes a bore 245, and rounded end 255 includes a bore 255. A pair of locking elements in the form of locking screws 230 are configured for insertion into bores 245, 255 to expand and lock rounded ends 244, 254 inside first socket 226 and second socket 228, respectively. Rounded ends 244, 254 are locked inside first and second sockets 226, 228 in the same way that screw heads 112 are locked inside first and second sockets 126, 128 in system 100. The rounded ends 244, 254 respectively include a plurality of petals 246, 256. Petals 246, 256 are separated from one another by a plurality of radially arranged slots 248, 258. One locking element 230 is configured for insertion between petals 246 to radially expand the petals into a locked condition when rounded end 244 of anchor element 240 is positioned inside first socket 226. The other locking element 230 is likewise configured for insertion between petals 256 to radially expand the petals into a locked condition when rounded end 254 of anchor element 250 is positioned inside second socket 228.

First and second anchor elements 240, 250 each provide a dual locking mechanism contained in a single component. That is, each anchor element 240, 250 contains two separate locking mechanisms integrally formed on a single unitary component. On first anchor element 240, for example, threaded end 242 is a first locking mechanism that is integrally formed with rounded end 244, the latter functioning as a second locking mechanism. Threaded end 242 couples anchor element 240 to bone screw assembly 210 and locks down first rod 260 in the bone screw assembly. In this condition, rounded end 244 projects out of bone screw assembly 210 in a position to receive cross connector 220. Cross connector 220 can be placed over the top of rounded end 244 by aligning socket 226 with the rounded end, and lowering the cross connector over the rounded end until the socket surrounds the rounded end as shown. Socket 228 of cross connector 220 cooperates with second anchor socket 250 in the same manner. In this configuration, cross connector 220 can be provided as an add-on to a pair of implanted pedicle screw assemblies. The first and second anchor elements 240, 250 replace standard set screws that are used to lock down the rods inside the implanted pedicle screw assemblies. As such, each of the first and second anchor elements 240, 250 is an adapter 240 that permits the cross connector to be added on to the implanted pedicle screw assemblies.

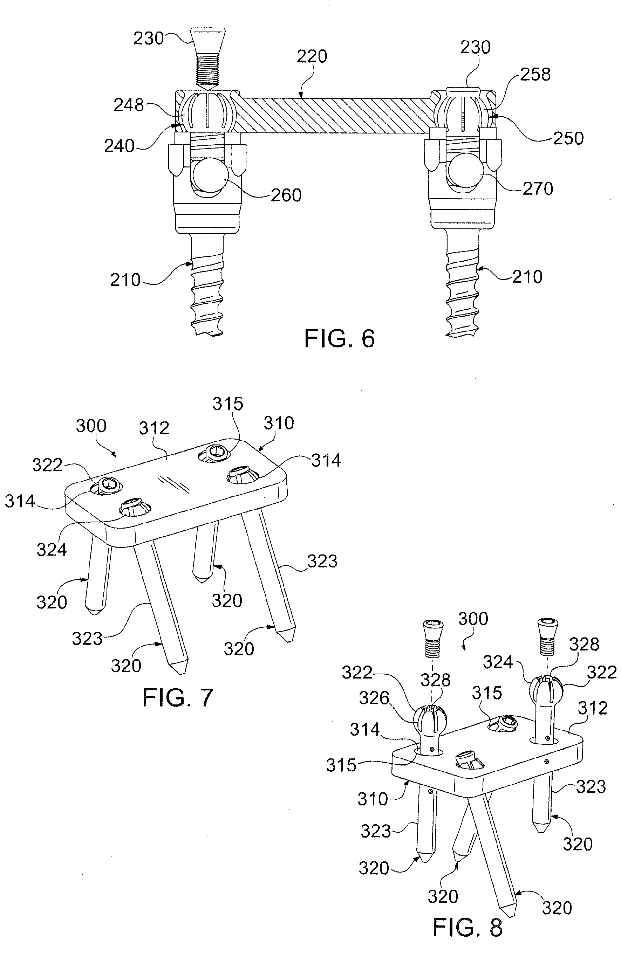

Referring to Figures 7 and 8, a low profile spinal stabilization system 300 is shown in accordance with another exemplary embodiment. System 300 includes a buttress style plate 310 that can be secured to the anterior column of the spine to provide additional stability to a spinal region. Plate 310 is substantially flat, and features a generally rectangular body 312 with four screw holes 314 extending through the body. Plate 310 is secured to vertebrae using four bone screws 320 that are inserted through screw holes 314. Bone screws 320 are lockable into screw holes 314 in the same manner that bone screws 110 in system 100 are lockable into sockets 126, 128. Each bone screw 320 includes a rounded head 322 and a threaded shank 323. Each head 322 has a plurality of radially expandable petals 324 separated from one another by a plurality of radially arranged slots 326, and an axial bore 328 extending between the petals. Bone screws 320 are insertable into screw holes 314 with screw heads 320 seated in the screw holes. Screw holes 314 form rounded seats 315 that slidably engage the rounded screw heads 320, so that the screws are polyaxially displaceable in the screw holes when in an unlocked condition. Locking elements in the form of locking screws 330 are insertable into the bores 328 of screw heads 322 to radially expand petals 324. Petals 324 are displaced outwardly and apply pressure to the seats 315, creating the necessary lockup conditions.

While preferred embodiments of the invention have been shown and described herein, it will be understood that such embodiments are provided by way of example only. Numerous variations, changes and substitutions will occur to those skilled in the art without departing from the scope of the invention. Accordingly, it is intended that the appended claims cover all such variations as fall within the scope of the invention.

## Claims

1. A low profile spinal stabilization system (100; 200) comprising:
an elongated fixation element (120; 220) comprising a first socket (126; 226) and a second socket (128; 228);
a first anchor element (110; 240) comprising a spherical end (112; 244) configured for insertion into the first socket (126; 226) of the elongated fixation element (120; 220), the spherical end (112; 244) of the first anchor element (110; 240) having a plurality of petals (114; 246) separated by a plurality of radially arranged slots (116; 248);
a second anchor element (110; 250) comprising a spherical end (112; 254) configured for insertion into the second socket (128; 228) of the elongated fixation element (120; 220), the spherical end (112; 254) of the second anchor element (110; 250) having a plurality of petals (114; 256) separated by a plurality of radially arranged slots (116; 258);
a first locking element (130; 230) configured for insertion between the petals (114) of the first anchor element (110; 240) and into the spherical end (112; 244) of the first anchor element (110; 240) to lock the spherical end (112; 244) of the first anchor element (110; 240) inside the first socket (126; 226) of the elongated fixation element (120; 220) when the spherical end (112; 244) of the first anchor element (110; 240) is positioned inside the first socket (126; 226); and
a second locking element (130; 230) configured for insertion between the petals (114) of the anchor element (110; 250) and into the spherical end (112; 254) of the second anchor element (110; 250) to lock the spherical end (112; 254) of the second anchor element (110; 250) inside the second socket (128; 228) of the elongated fixation element (120; 220) when the spherical end (112; 254) of the second anchor element (110; 250) is positioned inside the second socket (128; 228),
wherein the first socket (126; 226) is defined by a first end of the elongated fixation element (120; 220) and the second socket (128; 228) is defined by a second end of the elongated fixation element (120; 220), and
wherein the first locking element (130) is axially displaceable in the spherical head (112) of the first bone screw (110) between an unlocking position and a locking position,
**characterized in that** the petals of the first bone screw are expanded radially outwardly and have a first outer diameter in the unlocking position, and
**in that** the petals of the first bone screw are expanded further radially outwardly and have a second outer diameter in the locking position, the second outer diameter greater than the first outer diameter.

2. The low profile spinal stabilization system of claim 1, wherein at least one of the first anchor element (110; 240) and second anchor element (110; 250) comprises a bone screw (110).

3. The low profile spinal stabilization system of claim 1, wherein at least one of the first anchor element (240) and second anchor element (250) comprises an adapter (240; 250) for attaching the elongated fixation element (220) to a bone screw assembly (210).

4. The low profile spinal stabilization system of claim 3, wherein the adapter (240; 250) comprises a threaded end (242; 252) opposite the spherical end (244; 254), the threaded end (242; 252) configured for anchoring a rod (260; 270) in said bone screw assembly (210).

5. A low profile spinal stabilization system (100; 200) of claim 1,
wherein the spherical end of the first anchor element (110) is a spherical head (112),
wherein the spherical end of the second anchor element (110) is a spherical head, and
wherein the second locking element (130) configured to radially expand the petals (114) into a locked condition when the spherical head (112) of the second anchor element (110) is positioned inside the second socket.

6. The low profile spinal stabilization system of claim 1, wherein the first bone screw (110) is polyaxially rotatable inside the first socket (126) when the first bone screw (110) is inserted into the first socket (126) and the first locking element (130) is inserted into the spherical head (112) of the first bone screw (110) in the unlocking position, and wherein the first bone screw (110) is fixed relative to the first socket (126) when the first bone screw (110) is inserted into the first socket (126) and the first locking element (130) is inserted into the spherical head (112) of the first bone screw (110) in the locking position.

7. The low profile spinal stabilization system of claim 5, wherein at least one of the first socket (126) and second socket (128) comprises a spherical inner wall (127; 129) that conforms to the spherical head (112) of at least one of the first bone screw (110) and second bone screw (110).

8. The low profile spinal stabilization system of claim 5, wherein at least one of the first socket (126) and second socket (128) of the elongated fixation rod (120) is configured to receive substantially all of the spherical head (112) of at least one of the first bone screw (110) and second bone screw (110), and configured to receive substantially all of at least one of the first locking element (130) and second locking element (130).

9. The low profile spinal stabilization system of claim 5, wherein the first socket (126) comprises a bore (131), the bore (131) having a first end (131a), a second end (131b) opposite the first end (131a), and an inner diameter that is greatest at a section of the bore (131) located intermediate the first end (131a) and the second end (131b).

10. The low profile spinal stabilization system of claim 9, wherein the inner diameter of the bore (131) is largest at the midsection of the bore (131).

11. The low profile spinal stabilization system of claim 1, wherein at least one of the first socket and the second socket is cylindrical.

12. The low profile spinal stabilization system of claim 5, wherein the first anchor element (110) is a bone screw (110) and the second anchor element (110) is a bone screw (110),
wherein at least one of the first bone screw (110) and the second bone screw (110) comprises a shank (113), wherein the spherical head (112) of the at least one of the first bone screw (110) and the second bone screw (110) is formed of a first material, and the shank (113) is formed of a second material, the first material being more resilient and flexible than the second material.

## Patentansprüche

1. Niedrigprofil-Wirbelsäulenstabilisierungssystem (100; 200), umfassend:
ein langgestrecktes Fixationselement (120; 220), umfassend eine erste Fassung (126; 226) und eine zweite Fassung (128; 228);
ein erstes Ankerelement (110; 240), umfassend ein kugelförmiges Ende (112; 244), welches zum Einführen in die erste Fassung (126; 226) des langgestreckten Fixationselements (120; 220) ausgebildet ist, wobei das kugelförmige Ende (112; 244) des ersten Ankerelements (110; 240) eine Mehrzahl von blütenblattähnlichen Segmenten (114; 246) aufweist, welche über eine Mehrzahl von radial angeordneten Schlitzen (116; 248) voneinander getrennt sind;
ein zweites Ankerelement (110; 250), umfassend ein kugelförmiges Ende (112; 254), welches zum Einführen in die zweite Fassung (128; 228) des langgestreckten Fixationselements (120; 220) ausgebildet ist, wobei das kugelförmige Ende (112; 254) des zweiten Ankerelements (110; 250) eine Mehrzahl von blütenblattähnlichen Segmenten (114; 256) aufweist, welche über eine Mehrzahl von radial angeordneten Schlitzen (116; 258) voneinander getrennt sind;
ein erstes Verriegelungselement (130; 230), welches zum Einführen zwischen die blütenblattähnlichen Segmente (114) des ersten Ankerelements (110; 240) und in das kugelförmige Ende (112; 244) des ersten Ankerelements (110; 240) ausgebildet ist, um das kugelförmige Ende (112; 244) des ersten Ankerelements (110; 240) im Inneren der ersten Fassung (126; 226) des langgestreckten Fixationselements (120; 220) zu verriegeln, wenn das kugelförmige Ende (112; 244) des ersten Ankerelements (110; 240) im Inneren der ersten Fassung (126; 226) positioniert ist; und
ein zweites Verriegelungselement (130; 230), welches zum Einführen zwischen die blütenblattähnlichen Segmente (114) des Ankerelements (110; 250) und in das kugelförmige Ende (112; 254) des zweiten Ankerelements (110; 250) ausgebildet ist, um das kugelförmige Ende (112; 254) des zweiten Ankerelements (110; 250) im Inneren der zweiten Fassung (128; 228) des langgestreckten Fixationselements (120; 220) zu verriegeln, wenn das kugelförmige Ende (112; 254) des zweiten Ankerelements (110; 250) im Inneren der zweiten Fassung (128; 228) positioniert ist,
wobei die erste Fassung (126; 226) durch ein erstes Ende des langgestreckten Fixationselements (120; 220) definiert ist und die zweite Fassung (128; 228) durch ein zweites Ende des langgestreckten Fixationselements (120; 220) definiert ist und
wobei das erste Verriegelungselement (130) in dem kugelförmigen Kopf (112) der ersten Knochenschraube (110) zwischen einer Nicht-Verriegelungsposition und einer Verriegelungsposition axial verschieblich ist,
**dadurch gekennzeichnet, dass** die blütenblattähnlichen Segmente der ersten Knochenschraube in der Nicht-Verriegelungsposition radial nach außen expandiert sind und einen ersten Außendurchmesser aufweisen und
dass die blütenblattähnlichen Segmente der ersten Knochenschraube in der Verriegelungsposition radial weiter nach außen expandiert sind und einen zweiten Außendurchmesser aufweisen, wobei der zweite Außendurchmesser größer ist als der erste Außendurchmesser.

2. Niedrigprofil-Wirbelsäulenstabilisierungssystem nach Anspruch 1, wobei mindestens eines der Ankerelemente, welche sind das erste Ankerelement (110; 240) und das zweite Ankerelement (110; 250), eine Knochenschraube (110) umfasst.

3. Niedrigprofil-Wirbelsäulenstabilisierungssystem nach Anspruch 1, wobei mindestens eines der Ankerelemente, welche sind das erste Ankerelement (240) und das zweite Ankerelement (250), einen Adapter (240; 250) umfasst zum Anbringen des langgestreckten Fixationselements (220) an einer Knochenschraubenanordnung (210).

4. Niedrigprofil-Wirbelsäulenstabilisierungssystem nach Anspruch 3, wobei der Adapter (240; 250) ein mit einem Gewinde versehenes Ende (242; 252), welches dem kugelförmigen Ende (244; 254) gegenüberliegt, umfasst, wobei das mit einem Gewinde versehene Ende (242; 252) zum Verankern eines Stabs (260; 270) in der Knochenschraubenanordnung (210) ausgebildet ist.

5. Niedrigprofil-Wirbelsäulenstabilisierungssystem (100; 200) nach Anspruch 1, wobei das kugelförmige Ende des ersten Ankerelements (110) ein kugelförmiger Kopf (112) ist, wobei das kugelförmige Ende des zweiten Ankerelements (110) ein kugelförmiger Kopf ist und wobei das zweite Verriegelungselement (130) ausgebildet ist, die blütenblattähnlichen Segmente (114) in einen verriegelten Zustand zu expandieren, wenn der kugelförmige Kopf (112) des zweiten Ankerelements (110) im Inneren der zweiten Fassung positioniert ist.

6. Niedrigprofil-Wirbelsäulenstabilisierungssystem nach Anspruch 1, wobei die erste Knochenschraube (110) im Inneren der ersten Fassung (126) polyaxial rotierbar ist, wenn die erste Knochenschraube (110) in die erste Fassung (126) eingeführt ist und das erste Verriegelungselement (130) in den kugelförmigen Kopf (112) der ersten Knochenschraube (110) in der Nicht-Verriegelungsposition eingeführt ist, und wobei die erste Knochenschraube (110) relativ zu der ersten Fassung (126) festgelegt ist, wenn die erste Knochenschraube (110) in die erste Fassung (126) eingeführt ist und das erste Verriegelungselement (130) in den kugelförmigen Kopf (112) der ersten Knochenschraube (110) in der Verriegelungsposition eingeführt ist.

7. Niedrigprofil-Wirbelsäulenstabilisierungssystem nach Anspruch 5, wobei mindestens eine der Fassungen, welche sind die erste Fassung (126) und die zweite Fassung (128), eine kugelförmige Innenwand (127; 129) umfasst, welche dem kugelförmigen Kopf (112) mindestens einer der Knochenschrauben, welche sind die erste Knochenschraube (110) und die zweite Knochenschraube (110), entspricht.

8. Niedrigprofil-Wirbelsäulenstabilisierungssystem nach Anspruch 5, wobei mindestens eine der Fassungen, welche sind die erste Fassung (126) und die zweite Fassung (128), des langgestreckten Fixationsstabs (120) dazu ausgebildet ist, den kugelförmigen Kopf (112) mindestens einer der Knochenschrauben, welche sind die erste Knochenschraube (110) und die zweite Knochenschraube (110), im Wesentlichen ganz aufzunehmen, und dazu ausgebildet ist, mindestens eines der Verriegelungselemente, welche sind das erste Verriegelungselement (130) und das zweite Verriegelungselement (130), im Wesentlichen ganz aufzunehmen.

9. Niedrigprofil-Wirbelsäulenstabilisierungssystem nach Anspruch 5, wobei die erste Fassung (126) eine Bohrung (131) umfasst, wobei die Bohrung (131) ein erstes Ende (131a), ein dem ersten Ende (131a) gegenüberliegendes zweites Ende (131b) und einen Innendurchmesser, welcher an einem zwischen dem ersten Ende (131a) und dem zweiten Ende (131b) liegenden Abschnitt der Bohrung (131) am größten ist, aufweist.

10. Niedrigprofil-Wirbelsäulenstabilisierungssystem nach Anspruch 9, wobei der Innendurchmesser der Bohrung (131) an dem Mittelabschnitt der Bohrung (131) am größten ist.

11. Niedrigprofil-Wirbelsäulenstabilisierungssystem nach Anspruch 1, wobei mindestens eine der Fassungen, welche sind die erste Fassung und die zweite Fassung, zylinderförmig ist.

12. Niedrigprofil-Wirbelsäulenstabilisierungssystem nach Anspruch 5, wobei das erste Ankerelement (110) eine Knochenschraube (110) ist und das zweite Ankerelement (110) eine Knochenschraube (110) ist,
wobei mindestens eine der Knochenschrauben, welche sind die erste Knochenschraube (110) und die zweite Knochenschraube (110), einen Schaft (113) umfasst, wobei der kugelförmige Kopf (112) der mindestens einen der Knochenschrauben, welche sind die erste Knochenschraube (110) und die zweite Knochenschraube (110), von einem ersten Material gebildet ist und der Schaft (113) von einem zweiten Material gebildet ist, wobei das erste Material elastischer und flexibler ist als das zweite Material.

## Revendications

1. Système de stabilisation vertébrale à profil bas (100 ; 200) comprenant :
un élément de fixation allongé (120 ; 220) comprenant une première douille (126 ; 226) et une seconde douille (128 ; 228) ;
un premier élément d'ancrage (110 ; 240) comprenant une extrémité sphérique (112 ; 244) configurée pour l'insertion dans la première douille (126 ; 226) de l'élément de fixation allongé (120 ; 220), l'extrémité sphérique (112 ; 244) du premier élément d'ancrage (110 ; 240) ayant une pluralité de pétales (114 ; 246) séparés par une pluralité de fentes agencées de manière radiale (116 ; 248) ;
un second élément d'ancrage (110 ; 250) comprenant une extrémité sphérique (112 ; 254) configurée pour être insérée dans la seconde douille (128 ; 228) de l'élément de fixation allongé (120 ; 220), l'extrémité sphérique (112 ; 254) du second élément d'ancrage (110 ; 250) ayant une pluralité de pétales (114 ; 256) séparés par une pluralité de fentes agencées de manière radiale (116 ; 258) ;
un premier élément de blocage (130 ; 230) configuré pour être inséré entre les pétales (114) du premier élément d'ancrage (110 ; 240) et dans l'extrémité sphérique (112 ; 244) du premier élément d'ancrage (110 ; 240) afin de bloquer l'extrémité sphérique (112 ; 244) du premier élément d'ancrage (110 ; 240) à l'intérieur de la première douille (126 ; 226) de l'élément de fixation allongé (120 ; 220) lorsque l'extrémité sphérique (112 ; 244) du premier élément d'ancrage (110 ; 240) est positionnée à l'intérieur de la première douille (126 ; 226) ; et
un second élément de blocage (130 ; 230) configuré pour être inséré entre les pétales (114) de l'élément d'ancrage (110 ; 250) et dans l'extrémité sphérique (112 ; 254) du second élément d'ancrage (110 ; 250) pour bloquer l'extrémité sphérique (112 ; 254) du second élément d'ancrage (110 ; 250) à l'intérieur de la seconde douille (128 ; 228) de l'élément de fixation allongé (120 ; 220) lorsque l'extrémité sphérique (112 ; 254) du second élément d'ancrage (110 ; 250) est positionnée à l'intérieur de la seconde douille (128 ; 228),
dans lequel la première douille (126 ; 226) est définie par une première extrémité de l'élément de fixation allongé (120 ; 220) et la seconde douille (128 ; 228) est définie par une seconde extrémité de l'élément de fixation allongé (120 ; 220), et
dans lequel le premier élément de blocage (130) est axialement déplaçable dans la tête sphérique (112) de la première vis à os (110) entre une position de déblocage et une position de blocage,
**caractérisé en ce que** les pétales de la première vis à os sont expansés radialement vers l'extérieur et ont un premier diamètre externe dans la position de déblocage, et
**en ce que** les pétales de la première vis à os sont expansés davantage radialement vers l'extérieur et ont un second diamètre externe dans la position de blocage, le second diamètre externe étant supérieur au premier diamètre externe.

2. Système de stabilisation vertébrale à profil bas selon la revendication 1, dans lequel au moins l'un parmi le premier élément d'ancrage (110 ; 240) et le second élément d'ancrage (110 ; 250) comprend une vis à os (110).

3. Système de stabilisation vertébrale à profil bas selon la revendication 1, dans lequel au moins l'un parmi le premier élément d'ancrage (240) et le second élément d'ancrage (250) comprend un adaptateur (240 ; 250) pour fixer l'élément de fixation allongé (220) à un ensemble de vis à os (210).

4. Système de stabilisation vertébrale à profil bas selon la revendication 3, dans lequel l'adaptateur (240 ; 250) comprend une extrémité filetée (242 ; 252) opposée à l'extrémité sphérique (244 ; 254), l'extrémité filetée (242 ; 252) étant configurée pour ancrer une tige (260 ; 270) dans ledit ensemble de vis à os (210).

5. Système de stabilisation vertébrale à profil bas (100 ; 200) selon la revendication 1,
dans lequel l'extrémité sphérique du premier élément d'ancrage (110) est une tête sphérique (112),
dans lequel l'extrémité sphérique du second élément d'ancrage (110) est une tête sphérique, et
dans lequel le second élément de blocage (130) est configuré pour expanser radialement les pétales (114) dans une condition bloquée lorsque la tête sphérique (112) du second élément d'ancrage (110) est positionnée à l'intérieur de la seconde douille.

6. Système de stabilisation vertébrale à profil bas selon la revendication 1, dans lequel la première vis à os (110) peut tourner de manière polyaxiale à l'intérieur de la première douille (126) lorsque la première vis à os (110) est insérée dans la première douille (126) et le premier élément de blocage (130) est inséré dans la tête sphérique (112) de la première vis à os (110) dans la position de déblocage, et dans lequel la première vis à os (110) est fixée par rapport à la première douille (126) lorsque la première vis à os (110) est insérée dans la première douille (126) et que le premier élément de blocage (130) est inséré dans la tête sphérique (112) de la première vis à os (110) dans la position de blocage.

7. Système de stabilisation vertébrale à profil bas selon la revendication 5, dans lequel au moins l'une parmi la première douille (126) et la seconde douille (128) comprend une paroi interne sphérique (127 ; 129) qui est conforme à la tête sphérique (112) d'au moins l'une parmi la première vis à os (110) et la second vis à os (110).

8. Système de stabilisation vertébrale à profil bas selon la revendication 5, dans lequel au moins l'une parmi la première douille (126) et la seconde douille (128) de la tige de fixation allongée (120) est configurée pour recevoir sensiblement la totalité de la tête sphérique (112) d'au moins l'une parmi la première vis à os (110) et la seconde vis à os (110), et configurée pour recevoir sensiblement la totalité d'au moins l'un parmi le premier élément de blocage (130) et le second élément de blocage (130).

9. Système de stabilisation vertébrale à profil bas selon la revendication 5, dans lequel la première douille (126) comprend un alésage (131), l'alésage (131) ayant une première extrémité (131a), une seconde extrémité (131b) opposée à la première extrémité (131a), et un diamètre interne qui est le plus grand au niveau d'une section de l'alésage (131) positionnée entre la première extrémité (131a) et la seconde extrémité (131b).

10. Système de stabilisation vertébrale à profil bas selon la revendication 9, dans lequel le premier diamètre de l'alésage (131) est le plus grand au niveau de la section centrale de l'alésage (131).

11. Système de stabilisation vertébrale à profil bas selon la revendication 1, dans lequel au moins l'une parmi la première douille et la seconde douille, est cylindrique.

12. Système de stabilisation vertébrale à profil bas selon la revendication 5, dans lequel le premier élément d'ancrage (110) est une vis à os (110) et le second élément d'ancrage (110) est une vis à os (110),
dans lequel au moins l'une parmi la première vis à os (110) et la seconde vis à os (110) comprend une tige (113), dans lequel la tête sphérique (112) d'au moins l'une parmi la première vis à os (110) et la seconde vis à os (110), est formée avec un premier matériau, et la tige (113) est formée avec un second matériau, le premier matériau étant plus résilient et flexible que le second matériau.
